Europäisches Patentamt

European Patent Office

Office européen des brevets

(19)

(11) **EP 0 637 593 B1**

(12) **EUROPEAN PATENT SPECIFICATION**

(45) Date of publication and mention
of the grant of the patent:
**28.02.2001 Bulletin 2001/09**

(51) Int Cl.[7]: **C07K 16/46**, C07K 16/28,
A61K 39/395

(21) Application number: **94111346.6**

(22) Date of filing: **21.07.1994**

(54) **Bispecific trigger molecules recognizing lymphocyte antigen CD2 and tumor antigens**

Bispezifische Auslösemoleküle, die das Lymphozytantigen CD2 und Tumorantigene erkennen

Molécules de déclenchement bispécifiques pour reconnaître l'antigène lymphocytaire CD2 et des antigènes tumorales

(84) Designated Contracting States:
**AT BE CH DE DK ES FR GB GR IE IT LI LU NL PT SE**

(30) Priority: **02.08.1993 EP 93112330**

(43) Date of publication of application:
**08.02.1995 Bulletin 1995/06**

(73) Proprietor: **MERCK PATENT GmbH**
**64293 Darmstadt (DE)**

(72) Inventors:
• **Strittmatter, Wolfgang, Dr.**
**D-64372 Ober-Ramstadt (DE)**
• **Jäggle, Carlota-Silvia**
**D-64289 Darmstadt (DE)**
• **Meuer, Stefan, Prof. Dr.**
**D-69120 Heidelberg (DE)**
• **Shraven, Burkhart, Dr.**
**D-69118 Heidelberg (DE)**
• **Wild, Martin**
**D-69124 Heidelberg (DE)**

(56) References cited:
**EP-A- 0 260 880        EP-A- 0 294 703**
**WO-A-91/03493**

• **THE JOURNAL OF IMMUNOLOGY, vol.147, no.1, 1 July 1991, BALTIMORE MD, USA pages 60 - 69 A. TUTT ET AL. 'Trispecific F(ab')3 derivatives thar use cooperative signaling via the TcR/CD3 complex and CD2 to activate and redirect resting cytotoxic T cells.'**
• **IMMUNOBIOLOGY, vol.189, no.1-2, 30 September 1993, STUTTGART, GERMANY page 165 M. WILD ET AL. 'T cell targeting by anti-CD2 x anti-EGF-R bispecific antibodies.'**
• **IMMUNOBIOLOGY, vol.189, no.1-2, 30 September 1993, STUTTGART, GERMANY pages 155 - 156 C. JÄGGLE ET AL. 'Bispecific antibodies for targeted cellular cytotoxicity.'**
• **INTERNATIONAL JOURNAL OF CANCER, vol.55, no.3, 30 September 1993, GENEVA, SWITZERLAND pages 465 - 470 H. BERNHARD ET AL. 'Induction of tumor-cell lysis by bi-specific antibody recognizing ganglioside GD2 and T-cell antigen CD3.'**

EP 0 637 593 B1

## Description

The present invention relates to novel bispecific antibody fragments which recognize lymphocyte CD2 antigen and any variable tumor antigen. Preferably, the antigenous determinant of the epidermal growth factor-receptor (EGF-R) is used as tumor antigen. Moreover, the invention relates to two new monoclonal antibodies termed AICD2.M1 and AICD2.M2, and a combined positive effect of them on tumor lysis, whereby at least one of them is a bispecfic antibody fragment. The antibodies and antibody fragments can be used successfully in tumor therapy and diagnostics.

T lymphocytes are probably the most efficient components of the immune system when tumor cells are to be eliminated. However, most cancer patients do not have significant numbers of cytotoxic T lymphocytes (CTL) specifically reactive to their tumor cells (e.g. Knuth et al. 1984, Proc. Natl. Acad. Sci. (USA) 81, 3511). In order to activate the full lytic potential of CTL all these cells have to be directed towards tumor cells for which they have no natural specificity. T cells can be activated to proliferation, cytokine secretion and cytolytic activity by binding of monoclonal antibodies to certain membrane antigens on the surface of these cells.
Bispecific antibodies recognizing tumor-associated antigens with one binding arm and T cell markers with the other one have been showed to bridge monospecific CTL and malignant cells (e.g. Staerz et al. 1985, Nature 314, 628).
T cell activation by these artificial antibodies may occur through the T cell receptor (TCR)/ CD3 complex, in which the TCR is responsible for the antigen recognition and the CD3 epitope for the transduction of signals generated by the TCR-antigen interaction ( e.g. see review article of H. Nelson, Cancer Cells, May 1991, 163, and references cited herein).

Effector cell activation may also occur via the CD2 antigen, a glycoprotein which is present on T lymphocytes and natural killer (NK) cells (e.g. Hunig et al. 1987, Nature 326, 298). CD2 supports the interaction of the effector cell with the natural ligand LFA3 (CD58) on the target cell. Three functionally important epitopes (T11.1, T11.2 and T11.3) have been identified on CD2. In contrast to the CD3 antigen which undergoes modulation upon antibody binding there is no report sofare on CD2 modulation after targeting of CD2 with antibody. Earlier work using anti-CD2 antibody for activation has shown that a two-step activation via the CD2 antigen seems realistic. Synergistically acting anti-CD 2-specificities that have been used for activation sofare are characterized by antibodies such as anti T11.2 plus anti-T11.1 or T11.2 plus anti-TI 1.3. It has been reported that T11.3 is a *cryptic* epitope which becomes accessible after activation by anti T11.2. Most of the antibodies used sofare do compete with the natural CD2 ligand LFA-3 for binding (e.g. Bolhius et el. 1991, Cancer Immunol. Immunther. 34, 1, and references cited herein).

European patent application 294 703 (Schlossman et al.)discloses bifunctional anti CD-2 antibodies ($T11_2$ and $T11_3$) in order to address the alternative pathway of T-cell activation. According to Schlossman et al. the T11 epitopes are not expressed on resting, none activated T cells. Therefore, the bispecific antibodies described may only useful for targeting activated killer cells (NK, T cells). This bispecific antibody concept was based on the idea, that it would be possible to target and bind the bispecific antibody first to the tumor target and then active the T cells located a the target side in order to get bridging of tumor-target cell and killing via the alternative pathway. The bispecific antibodies of Schlossman crosslink target and effector cells however do not activate effector mechanisms. The activation is contributed by cytokines.

Glennie et al. (J. Immunol. 1991; 147: 60-9) and others have demonstrated that the simultaneous application of cross-linked antibodies directed to T cell receptor complex and several other T cell molecules, such as CD2, CD4, CD5, and CD8 can be used to activate resting T cells. In order to generate a more defined construct they have developed a method to synthesize trispecific F(ab')3 derivatives. These derivatives carrying two antibody arms binding to T lymphocytes and one single Fab' arm to target tumor cells have as well been described in WO 91/03493 by the same author. The two different CD2 epitopes involved in the application have been targeted with OKT11 and and an addtional antibody called GT2. The authors considered the antibodies to be equivalent to the $T11_1$ and $T11_3$ antibodies which have been published by Schlossman et al. and have been shown to activate lymphocyte via the alternative pathway of T-cell activation as described and used above. Using a (anti-T11(1) x anti-T11(3) x anti-CD37) trispecific F(ab')3 derivative in a therapeutic setting would mean, that the antibody binds to all CD2 positive NK and T cell and would induce a widespread lymphocyte activation, thus generating severe side effects.

Therefore, it was the aim of the present invention to develop new bispecific antibodies deriving from anti-tumor antibodies and a pair of synergistically acting anti-CD2 antibodies which are capable of delivering cytotoxicity after a two-step activation while leaving the normal cross-talk of cells via CD2/LFA3 uneffected.

It has been found that the new bispecific antibodies according to the invention have the following favorable properties: strong avidity to the tumor cell, strong avidity to T cells and NK cells, non- competitive against LFA3, no synergism with LFA3, no receptor modulation, high NK- and T cell specificity, effectiveness only via a two-step activation, that means that one bispecific antibody as such has no or only a marginal influence on T cell activation and tumor cell lysis, respectively.

Object of the invention is therefore a bispecific molecule, useful for lysis of tumor cells, comprising antibody determinants X and Z, wherein X is specific for an epitope on a tumor antigen, and Z is specific for an epitope of antigen CD2, wherein Z is selected from a monoclonal antibody designated as AICD2.M1, obtainable from hybridoma cell line 1 H 10 (DSM ACC2118) and another monoclonal antibody designated as AICD2.M2, obtainable from hybridoma cell line 7 D 3 (DSM ACC2119).

AICD2.M1 and AICD2.M2 are new monoclonal antibodies which were obtained by immunization of mice with genetically engineered CD2 antigen and isolation and purification of the antibodies from suitable mouse hybridom cells (details see examples) and which are highly specific to CD2 antigen of T- and NK cells.

Object of the present invention is, therefore, a monoclonal antibody termed AICD2.M1, recognizing CD2 antigen, obtainable by isolation from the cell line 1 H 10 deposited under accession no. **DSM ACC 2118.**

Object of the present invention is also a monoclonal antibody termed AICD2.M2, recognizing CD2 antigen, obtainable by isolation from the cell line 7 D 3 deposited under accession no. **DSM ACC 2119**.

The new cell lines producing said antibodies were deposited at "Deutsche Sammlung für Mikroorganismen" (DSM, Braunschweig, FRG) according to the Budapest Treaty on February 23, 1993.

The invention also relates to monoclonal antibodies which are natural or artificial variants or mutants of AICD2.M1 and AICD2.M2, whereby genetically modified or engineered, preferrably humanized and chimeric versions are included.

A bispecific molecule according to the invention comprises a binding arm of AICD2.M1 or AICD2.M2 and another binding arm of an antibody which recognizes any tumor antigen. The choice of the anti-tumor antigen has no significant influence on the effectiveness of the bispecific antibody regarding T cell activation and tumor cell lysis. In a preferred embodiment of the invention said second binding arm is represented by a binding arm of the monoclonal anti-tumor antibody MAb 425 or MAb 361, whereby, according to the invention, modified, preferrably humanized or chimeric versions and genetically engineered minimal fragments are included.

It is an object of the invention, therefore, to provide a bispecific antibody fragment as defined above and in the claims, wherein X is an antibody determinant deriving from the monoclonal antibodies MAb 425 *or* MAb 361.

MAb 425 is a murine monoclonal antibody raised against the well known human A431 carcinoma cell line (ATCC CRL 1555), binds to a polypeptide epitope of the external domain of the human EGF-R, and inhibits the binding of EGF. MAb 425 (ATCC HB 9629) was found to mediate tumor cytotoxicity in vitro and to suppress tumor cell growth of epidermoid and colorectal carcinoma-derived cell lines in vitro (Rodeck et al. 1987, Cancer Res. 47, 3692). Humanized and chimeric versions of MAb 425 have been disclosed in WO 92/15683.

MAb 361 is an IgG2a murine monoclonal antibody (ATCC HB 9325) and binds specifically to the ganglioside GD2 antigen and GD3 antigen. These gangliosides are greatly enriched in melanoma tumors (EP 0280 209, US SN 016902).

MAb 361 shows a significant level of cytotoxicity in vitro against tumor cells expressing GD2 antigen (Thurin et al. 1987, Cancer Res. 47, 1229).

The bispecific antibodies according to the invention are fragments of whole antibodies, for example, F(ab')2 molecules or miniantibodies (Fv molecules), preferably F(ab')2 molecules. In contrast to whole antibodies fragments such as F(ab')2 and miniantibodies may penetrate into the tumor mass of solid tumors more easily and reveal their cytotoxicity and power of cell lysis within the tumor. Moreover, F(ab')2 molecules and miniantibodies deriving from murine sources normally show a reduced human anti-mouse antibody response (HAMA).

Therefore, it is a preferred object of this invention to provide a bispecific F(ab')2 molecule as defined above and in the claims, wherein the combination of determinants X and Z is one of the following:

X is derived from Mab 425 and Z is derived from Mab AICD2.M1;
X is derived from Mab 425 and Z is derived from Mab AICD2.M2;
X is derived from Mab 361 and Z is derived from Mab AICD2.M1;
X is derived from Mab 361 and Z is derived from Mab AICD2.M2.

Various methods to produce bispecific antibodies based on complete antibodies or fragments have been described (e.g. see review article of: Brissinck et al. 1992, Drugs of the Future 17(11), 1003). One way to construct bispecific antibody fragments is to convert whole antibodies into (monospecific) F(ab')2 molecules by proteolysis, splitting these fragments into the Fab' molecules and recombine Fab' molecules with different specificity to bispecific F(ab')2 molecules (see for example EP 0179 872 B1).

Thus, it is an object of the present invention to provide a method of preparing a bispecific antibody F(ab')2 fragment useful for lysis of tumor cells comprising a first binding site to an epitope of a tumor cell and a second binding site to an epitope of CD2 antigen by enzymatical conversion of two different monoclonal antibodies, each comprising two identical L (light chain)-H (heavy chain) half molecules and linked by one or more disulfide bonds, into two F(ab')2 molecules, splitting each F(ab')2 molecule under reducing conditions into the Fab' thiols, derivatizing one of these Fab' molecules of each antibody with a thiol activating agent and combining an activated Fab' molecule bearing tumor specificity with a non-activated Fab' molecule bearing leucocyte specificity or vice versa in order to obtain the desired bispecific antibody F(ab')2 fragment, characterized in that monoclonal antibodies AICD2.M1 and AICD2.M2 are used as antibody recognizing leucocyte antigen.

As enzymes suitable for the conversion of an antibody into its F(ab')2 molecules pepsin and papain may be used. In some cases trypsin or bromelin are suitable. Pepsin, however, is preferrably used in the process of the present invention The conversion of the disulfid bonds into the free SH-groups (Fab' molecules) may be performed by reducing compounds, such as dithiotreitol (DTT), mercaptoethanole, and mercaptoethylamine. Thiol activating agents acoording to the invention which prevent the recombination of the thiol half-molecules, are 5,5'-dithiobis (2-nitrobenzoic acid) (DTNB), 2,2'-dipyridine disulfide, 4,4'-dipyridine disulfide or tetrathionate/sodiumsulfite (see also Raso et al. 1982, Cancer Res. 42, 457). Preferrably, DTNB is used in the process according to this invention.

The treatment with the thiol-activating agent is performed only with one of the two Fab' fragments. Principally, it makes no difference which one of the two Fab' molecules is converted into the activated Fab' fragment (e.g. Fab'-TNB). Generally, however, the Fab' fragment being more labile is modified with the thiol-activating agent. In the present case, the fragments bearing the anti-tumor specificity are a little bit more labile, and, therefore, preferrably used in the process. The conjugation of the activated Fab' derivative with the free hinge-SH groups of the second Fab' molecule to generate the bivalent F(ab')2 antibody occurs spontaneously at temperatures between 0 and 30°C. The yield of purified F(ab')2 antibody is 20 to 40% (starting from the whole antibodies).

As mentioned above, the invention includes also bispecific miniantibodies. Miniantibodies are antibody fragments comprising two single-chain Fv fragments (scFv fragments are usually genetically linked either as $V_H$-linker-$V_L$ or as $V_L$-linker-$V_H$) which are linked by autoaggregation or by fusing each of them at the C-terminus to a hinge region (optionally) to provide flexibility, and / or an amphipathic helix to achieve dimerization. The helix can be taken from a four-helix-bundle design or from a leucine zipper (e.g. Pack and Plückthun 1992, Biochem. 31, 1579). The preparation of miniantibodies as defined above, is disclosed, for example, in WO 93-00082.

The bispecific antibody fragments according to the invention were tested in vitro/ex vivo regarding following properties: capacity to bind to the specific tumor- and T cells, immunmodulation (T cell proliferation) and cytotoxicity (tumor cell lysis). The tests are described per se in the standard literature. Details and results are given in the examples.

The bispecific antibody fragments according to the invention can be administered to human patients for therapy. Therefore, it is an object of the invention to provide a pharmaceutical formulation comprising as active ingredient at least one bispecific antibody fragment as defined above and in the claims, associated with one or more pharmaceutically acceptable carrier, excipient or diluent therefore.

Typically the antibody fragments of this invention will be injected intravenously or parenterally. Generally, the dosage ranges for the administration of the bispecific antibody fragments are large enough to produce the desired tumor suppressing and tumor lysing effect. The dosage will depend on age, condition, sex and extent of the desease in the patient and can vary from 0.1 mg/kg to 200 mg/kg, preferably from 0.1 mg/kg to 100 mg/kg/dose in one or more doses administrations daily, for one or several days.

Preparations for parenteral administration includes sterile aqueous or non-aqueous solutions, suspensions, and emulsions. Examples of non-aqueous solvents are propylene glycol, polyethylene glycol, vegetable oils such as olive oils, and injectable organic esters such as ethyl oleate and other solvents known in the art which are suitable for these purposes. The bispecific antibodies of this invention can be used in a composition comprising a physiologically acceptable carrier. Examples of such suitable carriers are saline, PBS, Ringer's solution, or lactated Ringer's solution. Preservatives and other additives such as antibiotics, antioxidants, and chelating agents may also be present in the pharmaceutical formulations. The bispecific antibody fragments can also be conjugated according to known methods to cytokines such as IL-2 in order to support their cytotoxicity.

The methods of chemical linkage of antibody fragments (single-chain Fv and Fab fragments) with the aid of crosslinking agents are, for example, described by Segal and Snider 1989, Chem Immunol. 47, 179; Glennie et al. 1987, J. Immunol., 139, 2367; Brennan et al. 1985, Science, 229, 81 and PCT/US91/07283 (Romet-Lemonne and Fanger,).

The pharmaceutical formulations of the present invention are suitable for the treatment of all kinds of tumors, including melanomas, gliomas and carcinomas, as well as tumors of the circulating system and solid tumors. Preferred formulations comprise a bispecific antibody fragment, wherein one binding arm bears the binding sites of the monoclonal antibody MAb 425, or MAb 361.

The present invention discloses a very effective two-step activation of leucocytes by administration by two different antibodies or bispecific antibody fragments, respectively.

The two-step activation according to this invention means that, unless

(a) a first formulation comprising a bispecific antibody fragment having a defined tumor cell specificity and a CD2 binding site, is administered with

(b) a second formulation comprising an additional, different bispecific antibody fragment having a different CD2 binding site but the same tumor cell specificity, or a monoclonal antibody having a different CD2 binding site and no tumor specificity, or a fragment of said antibody,

the first bispecific antibody fragment is either ineffective or only marginally (20-40 % as compared with the two-step formulation) effective as a single agent.

Therefore, it is an object of the invention to provide a pharmaceutical kit of parts (A) comprising (i) a first pharmaceutical formulation (I) comprising a bispecific molecule as defined above and in claim 1 together with a pharmaceutically acceptable carrier, excipient or diluent, wherein Z is derived from AICD2.M2, and (ii) a second seperate pharmaceutical formulation (II) comprising MAb AICD2.M1 or a Fv or a F(ab')2 fragment thereof or a bispecific molecule as defined above and in claim 1, wherein Z is derived from MAb AICD2.M1. In this pharmaceutical kit of parts formulation (I) is based on MAb AICD2.M2 and formulation (II) on MAb AICD2.M1.

Alternatively, the invention relates to another kit of parts, wherein formulation (I) is based upon MAb AICD2.M1, whereas formulation (II) is based on MAb AICD2.M2. Thus, it is a further object of this invention to provide a pharmaceutical kit of parts (B) comprising (i) a first pharmaceutical formulation (I) comprising a bispecific molecule as defined above and in claim 1 together with a pharmaceutically acceptable carrier, excipient or diluent, wherein Z is derived from AICD2.M1, and (ii) a second seperate pharmaceutical formulation (II) comprising MAb AICD2.M2 or a Fv or a F(ab')2 fragment thereof or a bispecific molecule as defined above and in claim 1, wherein Z is derived from MAb AICD2.M2.

The differences between composition (A) and (B) are normally not very significant. In the case of MAb 425 it can be seen that the composition (B), wherein formulation (I) is based on MAb AICD2.M1 works better.

Differences in tumor binding sites within a composition ((A) or (B)) are also not very significant. The effectiveness of the pharmaceutical kits of parts according to the invention regarding binding capacity, immun-modulation (leucocyte proliferation) and cytotoxicity depends, however, on the influence of the binding sites of the bispecific antibody fragments which derive from the antibodies AICD2.M1 and AICD2.M2. Thus, the preferred embodiments of this invention show similar properties.

Differences in therapeutical effect regarding formulation (II) (alternatively whole antibody, monospecific antibody fragment or **bispecific antibody**) are also not very dramatical. However, formulations (II) comprising the suitable monospecific antibody fragment are preferred.

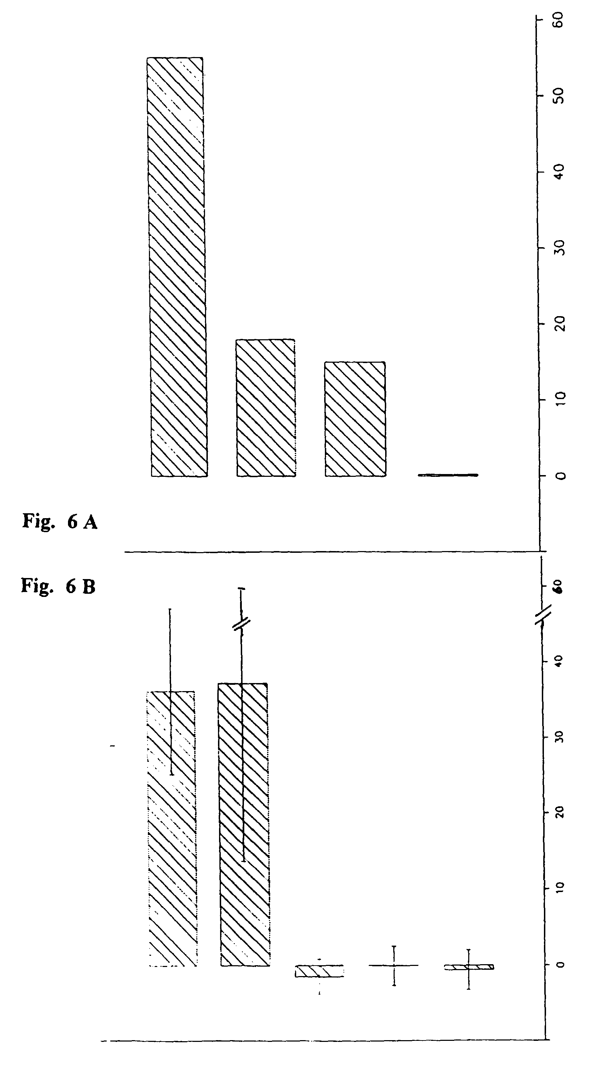

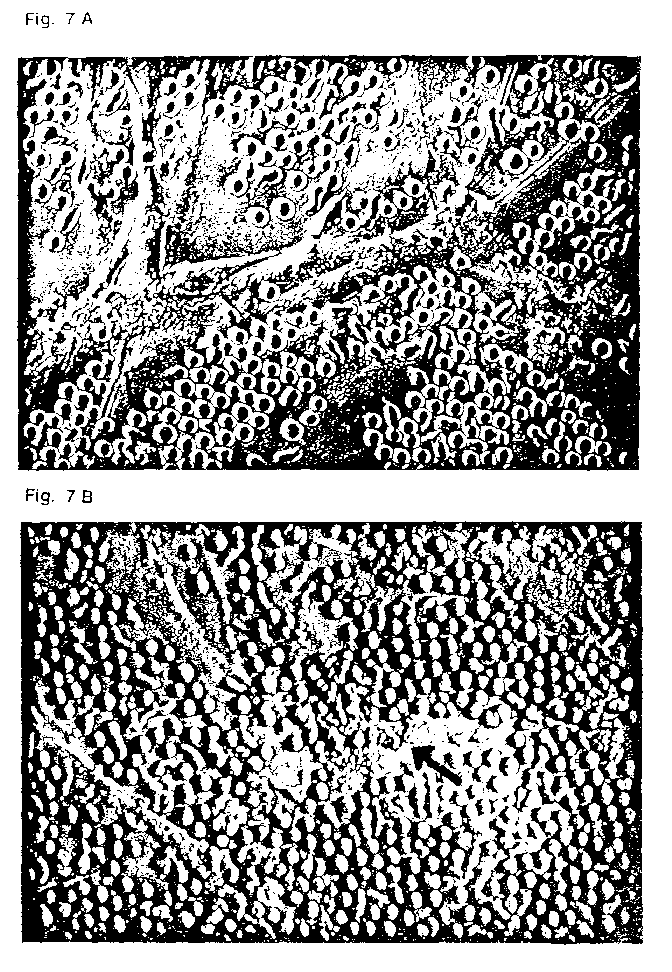

The pharmaceutical kit of parts is used as follows. Medical treatment is started by injecting formulation (I) of one of the pharmaceutical kit of parts according to the invention into the patient. The bispecific antibody binds according to its specificity not only to the surface of the tumor, but for reasons of its small size, it may also penetrate into the solid tumor mass. The administration of the bispecific antibody fragment according to the invention causes an enrichement of injected immunoglobulin in the local area of the tumor, but does not yet lead to a significant immune response. After an individual time period (dependent on extent of disease, type of tumor, condition, sex and age of the patient) of several hours to several days formulation (II) is adminstered and immunolgical responses ared induced immediately. Formulation (I) and (II) are preferrably administered in equimolar amounts. Ex vivo experiments show that autologous and allogeneic tumor-infiltrating lymphoctes (TILs) which are normally unable to induce significant cytotoxicity against the tumor (cell lysis) can be highly activated by said treatment (Fig. 6a,b, 7 ).

By means of a pharmaceutical kit of parts according to this invention it is also possible to purge ex vivo tumor cells in bone marrow preparations. In order to intensify the cytotoxic effect, it is possible to add exogenous T lymphocytes.

Therefore, it is finally an object of the present invention to provide a method for purging of tumor cells ex vivo in autologous bone marrow transplantation by means of a pharmaceutical kit of parts as defined above or in the claims, optionally in the presence of additional T-lymphocytes, whereby to begin with the cells are treated with formulation (I) and after that with formulation (II), optionally followed by a conventional purification step. The techniques of purging bone marrow ex vivo are well known in the art per se.

**Brief descripton of the figures:**

**Fig. 1:** Synthesis of **bispecificantibodies** (scheme).

**Fig. 2:** SDS-PAGE of antibody fragments and **bispecificantibodies**:

Lane 1: molecular weight marker,
Lane 2: **F(ab')2 of MAb AICD2.M1** before reduction with DTT,
Lane 3: **F(ab)2 of MAb 425** before reduction with DTT,

Lane 4: *F(ab)2 of MAb AICD2.M1* after reduction with DTT and derivatization with $DTN_3$,
Lane 5: *F(ab)2 of MAb 425* after reduction with DTT and derivatization with $DTN_3$,
Lane 6: *bispecific antibodies* before and
Lane 7: *bispecific antibodies* after purification.

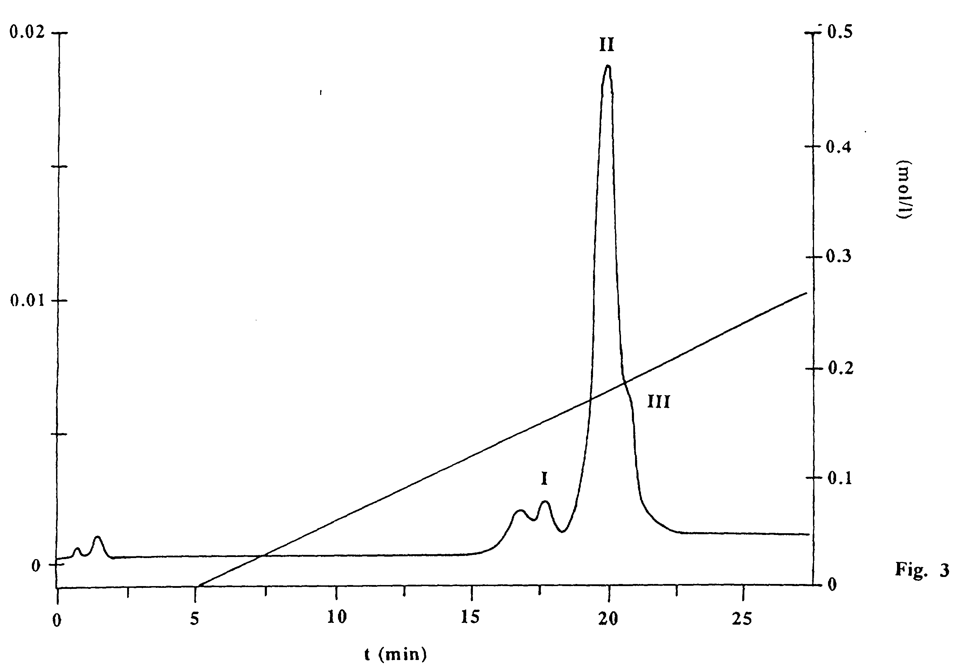

**Fig 3**: Chromatographic profile of *abispecificF(ab')2moleculeof Mab425andMAbAICD2.M1* on hydroxylapatite:

left vertical axis: optical density OD at 280 nm, right vertical axis: concentration phosphate buffer (pH = 7.0), horizontal axis: elution time (min). The bispecific antibody fragment is represented by peak II. Peaks I and III are minor contaminates.

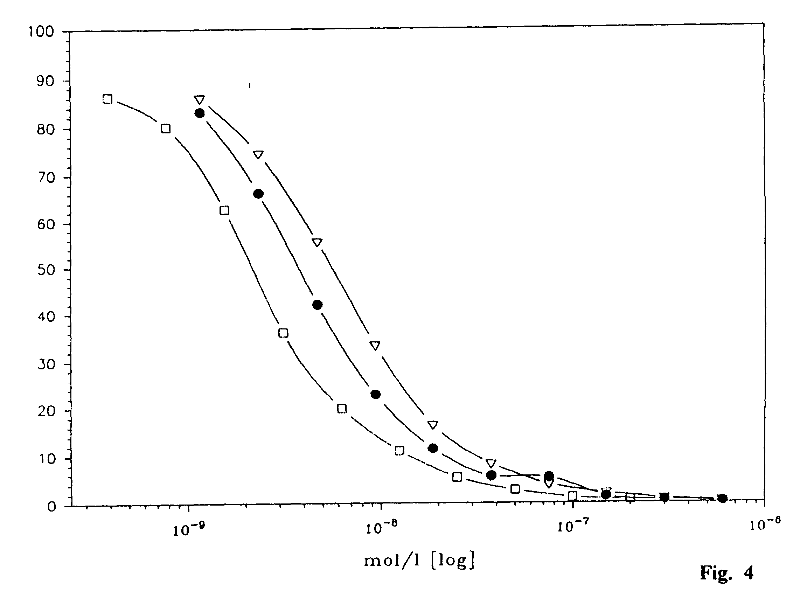

**Fig. 4:** Competetive binding of *bispecificantibodiesandMAbs* to EGF-R:

vertical axis: % absorbance at 490 nm, horizontal axis: concentration of antibody fragments in mol/l (logarithmic scale), biotin-labeled MAb 425 has been used to compete with unlabeled *F(ab')2 of Mab 425 or bispecific antibody* for binding to EGF-R;

- *bispecific F(ab')2 molecule of Mab 425 and MAb AICD2.M1*
- ▽ *bispecific F(ab')2 molecule of Mab 425 and MAb AICD2.M2*
- □ *F(ab')2 molecule of Mab 425*

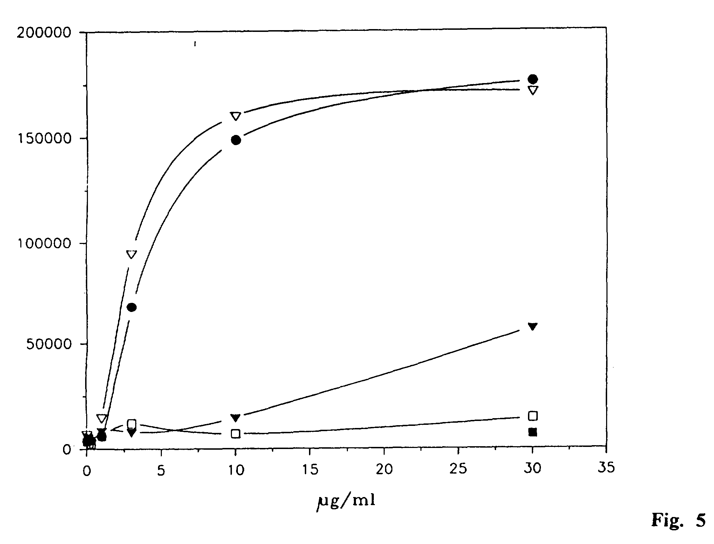

**Fig. 5**: Proliferation of human peripheral blood leucocytes (PBL):

vertical axis: % incorporation of 3H-thymidine, horizontal axis: concentration of antibody fragments (μg/ml);

- *bispecific F(ab')2 molecule of MAb 425 and MAb AICD2.M1 + F(ab')2 of MAb AICD2.M2*
- ▽ *bispecific F(ab')2 molecule of MAb 425 and MAb AICD2.M2 + F(ab')2 of MAb AICD2.M1*
- ▼ *F(ab')2 of MAb AICD2.M2*
- □ *F(ab')2 of MAb AICD2.M1*
- ■ *F(ab')2 of MAb 425*

**Fig. 6a:** *Tumor lysis induced by bispecific antibodies* allogeneic TILs as effector cells:

vertical axis: % cytotoxicity,
   horizontal axis:

   1 = *bispecific F(ab')2 molecule of MAb 425 and MAb AICD2.M1 + F(ab')2 of MAb AICD2.M2*
   2 = *F(ab')2 of MAb AICD2.M1*
   3 = *F(ab')2 of MAb AICD2.M2*
   4 = *F(ab')2 of MAb 425*

**Fig. 6b:** *Tumor lysis induced by bispecific antibodies* autologous TILs as effector cell :

vertical axis: % cytotoxicity,
   horizontal axis:

   1 = *bispecific F(ab')2 molecule of MAb 425 and MAb AICD2.M1 + F(ab')2 of MAb AICD2.M2*
   2 = *bispecific F(ab')2 molecule of MAb 425 and MAb AICD2.M2 + F(ab')2 of MAb AICD2.M1*
   3 = *F(ab')2 of MAb AICD2.M1*
   4 = *F(ab')2 of MAb AICD2.M2*
   5 = *F(ab')2 of MAb 425*

vertical lines on top of the bars: average deviation.

**Fig. 7:** *Tcelltargetingandtumorcelllysismediatedbybispecific antibodies :*

Figures 7 A and B show a coculture of tumor infiltrating lymphocytes (TILs) together with autologous malignant cells at an effector / target ratio of 40 / 1. Coculture was incubated for 4 hours without (Fig. 7 A, control) and in the presence of

**bispecific F(ab')2 molecule of MAb 425 and MAb AICD2.M1 + F(ab')2 of MAb AICD2.M2**

(20 µg/ml each) (Fig. 7 B). In the presence of CD2 **bispecific antibody** numerous T lymphocytes adhere and cluster to the autologous tumor cells subsequently killing the melanoma cells. The arrow in Fig. 7 B indicates a conjugate of TILs with destroyed melanoma cell. Coculture of TILs and melamoma cells were done in 24 well plates in culture medium containing 10% FCS. Micrographs were achieved with an inverse microscope (modified phase contrast, original magnification 100).

The following examples describe the invention in detail.

**EXAMPLE 1:**

Preparation of MAbs AICD2.M1 and AICD2.M2:

Balb/C mice were immunized four times within two weeks with genetically engineered CD2. The DNA sequence of human CD2 and its cloning has been described by Sayre et al. 1987, Proc. Natl. Acad. USA 84, 2941. The CD2 gene expression was done in the Baculovirus system according to methods known in literatur (Fraser 1992, Current Topics in Microbiol. Immunol. 158, 131, and references cited herein).

Spleen cells of immunized mice were isolated and fused with the myeloma cell line (Ag8.653, ATCC CRL 1580). Hybridomas were screened for specific monoclonal antibody production by testing the supernatants of growing hybridomas on human T lymphocytes (CD2 positive) by means of fluorescence-activated cell sorter (FACS). Positive hybridomas were recovered by the method of "limited dilution cloning". The supernatants of the selected hybridomas were investigated by means of immunoprecipitation and western blot analysis. The isolation of the selected monoclonal antibodies was achieved by purification of cultures of supernatants according to Ey et al. 1978, Immunochemistry 15, 429.

Functional analysis of the antibodies directed to the CD2 antigen were done by means of a proliferation test (incorporation of $^3$H-thymidine). Two antibodies selected from a series of monoclonal antibodies reacting with CD2 antigen strongly induced the proliferation of T cells. These monoclonal antibodies were designated as AICD2.M1 and AICD2.M2 and deposited at Deutsche Sammlung fur Mikroorganismen.

All used methods were achieved by standard procedures. Many of them are disclosed, for example, in "Antibodies, a Laboratory Manual", Harlow and Lane, 1988, Cold Spring Harbor, and references cited herein.

**EXAMPLE 2:**

Synthesis of **bispecific F(ab')2 molecule of MAb 425 and MAb AICD2.M1:**

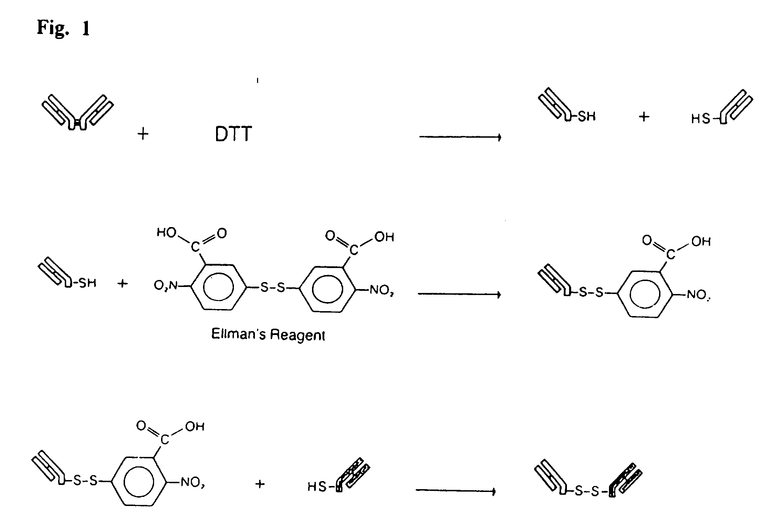

Synthesis started from 10mg of the antibody AICD2.M1 and 10 mg oft the antibody MAb 425. Antibodies used were treated by pre-activated papain-treatment (10 mg/ml) in citric acid buffer, 3mM EDTA, pH 5.5. After proteolysis F(ab')2 was recovered from the eluate of a **Protein-A-Sepharose®** column. The recovery of F(ab')2 is generally close to 100%. Both F(ab')2 fragments were individually converted into the Fab' fragment by treatment with 0.5mM DTT for 40 min at 30 C. After the reduction step surplus of DTT was removed and Fab' recovered via gelfiltration (Superdex® 200). In the ongoing process the Fab'-TNB derivative was generated by treating the MAb 425 fragment with 0.5mM Ellman's reagent (5,5'-dithio-bis-(2-nitro-benzoic acid) (DTNB)) at 4°C for 60 min. Surplus of DTNB was removed by gelfiltration. 6mg of each conjugation partner (Fab' - AICD2.M1 and Fab'-TNB MAb 425) was used for the final conjugation step by stirring the mixture of the conjugation partners at 4°C for 18 hours. Bispecific antibody was recovered from the reaction mixture by gelfiltration chromatography (Superdex® 200). The yield of the synthetic F(ab')2 preparation was in a range of 3-4mg ($\approx$ 30%). The sequenzes of operations involved in **bispecific antibody** synthesis have been summarized in Fig. 1.

**EXAMPLE 3:**

Synthesis of **bispecific F(ab')2 molecule of MAb 361 and MAb AICD2.M1:**

According to Example 2 a bispecific antibody was prepared starting from MAb AICD2.M1 and MAb 361. The yield of **bispecific antibody** was approximately 34%.

**EXAMPLE 4 :**

Synthesis of *bispecific F(ab')2 molecule of MAb 425 and MAb AICD2.M2:*

According to Example 2 a bispecific antibody was prepared starting from MAb AICD2.M2 and MAb 425. The yield of *bispecific antibody* was approximately 30%.

**EXAMPLE 5:**

Synthesis of *bispecific (scFv)2 molecule of MAb 425 and MAb AICD2.M1:*

Two single chain proteins consiting of the VL and VH domaines of AICD2.M 1 and MAb 425 have been prepared by molecular engineering (Winter et al. 1991, Nature 349, 293-299). The expression plasmids were derived from pHEN1 (Hoogenboom et al. Nucleic Acid Res. 19, 4133-4137) which contains the structural genes for the VL and VH domains derived from either the anti-EGF-R murine hybridoma (MAb 425) or the anti-CD2 murine hybridoma (AICD2.M1). The pelB leader sequenc was used to mediate periplasmatic secretion in E. coli. scFVs were purified by affinity chromatography and used for the construction of bispecific scFv. Chemical cross-linking of EGF-R and CD2-specific scFvs has been done with the help of the reagent 2-Iminothiolane and the heterobifunctional cross-linker SPDP. Starting from 10 mg of *scFv molecule of MAb 425* we have approached the statistical incoorporation of one molecule of 2-pyridyl-disulfide per scFv molecule via SPDP. Purification of the derivative was performed with gelfiltration chromatography. Introduction of reactive -SH group into the *scFv molecule of MAb AICD2.M1* was obtained by derivatization of 10 mg scFv with 2-Iminothiolane. Purification of the derivative was performed with gelfiltration chromatography. Equimolar quantities of 2-pyridyl-disulfide activated *scFv molecule of MAb 425* and the 2-Iminothiolane modified *scFv molecule of MAb AICD2.M1* were coupled together at neutral pH. The coupling reaction was followed by monitoring the liberation of pyridine 2-thione. The final bispecific product was recovered by gelfiltration chromatography with a yield of about 5%.

**EXAMPLE 6 :**

Charcterization of the bispecific antibodies:

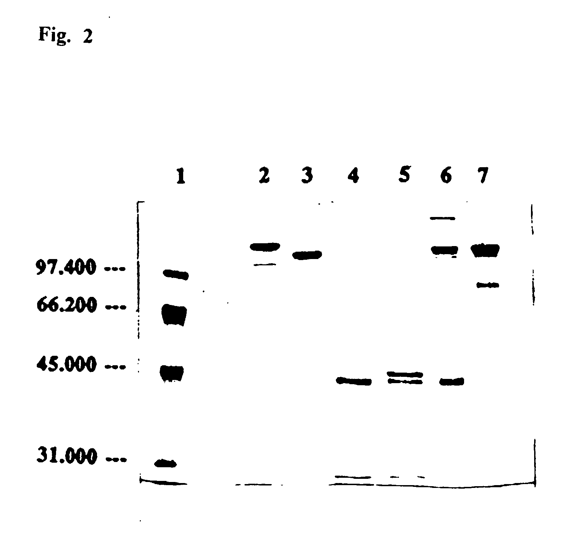

The individual steps of synthesis *of bispecific antibodies* have been monitored by SDS-PAGE according to usual known techniques. The intermediates of the chemical synthesis and the final bispecific products are shown in Fig. 2. In addition to SDS-PAGE analysis purity and homogeneity of *bispecific antibody* has been assessed by chromatography on a hydroxylapatide column. Elution of *bispecific antibody* was performed with a gradient of phosphate buffer (pH = 7.0, 0 - 0.3 mol/l). Fig. 3 showes the chromatographic profile of a *bispecific F(ab')2 molecule of MAb 425 and MAb AICD2.M1*. The *bispecific antibody* is represented by the major peak II the smaller peaks I and III are minor contaminates.

**EXAMPLE 7 :**

Binding properties *of bispecific F(ab')2 molecule of MAb 425 and MAb AICD2.M1* to EGF-R:

The binding properties of the bispecific antibody have been compared by competitive ELISA assay. In short: Biotin-labeled MAb 425 has been used to compete with unlabeled antibody or *bispecific antibodies* for binding to EGF-R. EGF-R was isolated from A 431 cells (ATCC CRL 1555) according to kown methods (e.g. Harlow, Lane: "Antibodies, A Laboratory Manual", Cold Spring Harbor, 1988). A 431 cells are CD2 negative. Detection was done after incubation with POD-conjugated streptavidin and substrate. From the data inhibition curves have been constructed (Fig. 4). The inhibition curves for both *bispecific antibodies* where shifted to the right, indicating loss in affinity due to change from divalent to monovalent binding.

**EXAMPLE 8 :**

Binding properties of *bispecific F(ab')2 molecule of MAb 425 and MAb AICD2.M2* to EGF-R:

According to Example 8 the binding properties of the bispecific antibody were investigated (Fig. 4). Both *bispecific antibodies* were of comparable avidity towards their antigen target.

**EXAMPLE 9 :**

Binding properties of bispecific and monospecific antibody fragments to CD2 antigen:

Jurkat cells (human acute T cell leukemia, CD2, CD3 positive, EGF-R negative; ATCC TIB 152) were incubated (1 x $10^6$ cells/sample) with logarithmical dilutions of the bispecific/monospecific antibody fragment for 15 minutes at about 4°C. Cells were then washed and incubated with goat-antimouse-kappa-FITC as a second step antiserum. Cells were washed again and immunofluorescence was analysed with a FACStar plus®. The FACStar plus ® with single laser exitation and list mode data acquisition was used to analyse antibody staining of living cells. The specific fluorescence intensities of the whole cell population and the percentage of antibody reactive cells were determined. Fluorescence distributions of cells stained with the second step antiserum only served as negative controls.
In the following table half saturating concentrations (conc. $_{1/2\ sat.}$) as measure of binding strength of the different bispecific/monospecific antibody fragments on Jurkat cells are indicated. Values for conc. $_{1/2\ sat}$ were read from the titration curves (fluorescence intensity (linear presentation) versus antibody concentration) and thus are rough estimations.

| antibody fragment | conc. $_{1/2\ sat}$ |
|---|---|
| *F(ab)2 of MAb AICD2.M1* | 3 x $10^8$ |
| *bispecific F(ab')2 molecule of MAb 425 and MAb AICD2.M1* | >1 x $10^7$ (no satur.) |
| *bispecific F(ab')2 molecule of MAb 361 and MAb AICD2.M1* | 1 x $10^7$ (no satur.) |
| *F(ab')2 of MAb AICD2.M2* | 2 x $10^8$ |
| *bispecific F(ab')2 molecule of MAb 425 and MAb AICD2.M2* | >1 x $10^7$ (no satur.) |
| *F(ab')2 of MAb 425* | negative |

**EXAMPLE 10 :**

Proliferation of leucocytes:

Peripheral blood mononuclear leukocytes were co-cultured with irradiated (30Gy) autologous tumor cell in medium (RPMI 1640) supplemented with I1-2 (20 U/ml) and I1-4 (1000 U/ml). Responder cells were weekly restimulated with autologous tumor cells.
Freshly prepared human peripheral blood leucocytes (huPBL) from healthy blood donors or melanoma patients were cultured in 96 well flat-bottom microtiter plates in a final volume of 200 µl at a density of 1 x $10^5$ cells/well. Cells were incubated with monospecific or monospecific + bispecific antibody fragments in indicated concentrations. After 72 hours the cells were pulsed with 0.5 µCi $^3$H-thymidine, incubated overnight, and harvested. The incorporation of $^3$H-thymidine was measured by liquid scintillation β-plate counting, and the results expressed as the average cpm. The results for antibody fragments deriving from MAb 425 can be seen in detail from Fig. 5. Similar results were obtained using antibody fragments deriving from MAb 361. Combined administration of antibody fragments cause a significant induction of proliferation in each of these cases.

**EXAMPLE 11 :**

***Tumorlysis induced by bispecific antibody :***

The cell line C8161, a highly invasive and spontanously metastatic, EGF-R positive human melanoma cell line (Welch et al. 1991, Int. J. Cancer 47, 227, and references cited herein) was used as target for EGF-R dependent targeting by the allogeneic and autologous tumor infiltrating T lymphocytes (TILs). Other EGF-R positive human melanoma cell lines can also be used. TIL bulk culture was established from a melanoma specimen. Culture conditions of tumor cells and TILs have been described previously (e.g. Shimizu et al. 1991, Cancer Res. 51, 6153).
Production of stable CTL clones has been done with MLTC responder cells under limiting dilution condititons. MTLC responder T cells were seeded at one cell per well in 96-well plates in medium supplemented with 11-2 and IL-4 together with autologous stimulatory cells and autologous EBV-transformed B cells as feeder cells. Colonies were weekly restimulated with autologous tumor cells and feeder cells.
T lymphocytes from fresh tumor biopsies were activated with immobilized anti-CD3 antibody and expanded in RPMI supplemented with I1-2 and I1-4. In vitro Cytotoxicity assays were performed using $^{51}$Cr-labeled tumor cells. Target cells were labeled with $^{51}$Cr (100 mCi/$10^7$ cells) for 1 hrs and washed 3 times. A constant number of labeled cells (2 x $10^3$/well) were co-incubated with monospecific or bispecific antibody or a combination of them together with effector TILs in an effector/target ratio of 7/1. CTL were serially diluted in triplicates in 100ml of medium in 96-well microtiter plates. After adding labeled target cells the plates were incubated 4hrs at 370C in 10% $CO_2$. The assays were stopped by centrifugation. The supernatantes were collected and radioactivity was measured with a g-counter. Percent specific $^{51}$Cr-release was calculated according to the formula:

$$\% \text{ specific } {}^{51}\text{Cr-release} = \frac{100 \text{ (experimental release - spontaneous release)}}{\text{(maximum release - spontaneous release)}}$$

The results for antibody fragments deriving from MAb 425 can be seen in detail from Fig. 6a, 6b and Fig. 7. Similar results were obtained using antibody fragments deriving from MAb 361. Combined administration of antibody fragments cause a significant induction of tumor lysis in each of these cases.

**Claims**

1. A bispecific molecule, useful for lysis of tumor cells, comprising antibody determinants X and Z, wherein X is specific for an epitope on a tumor antigen, and Z is specific for an epitope of antigen CD2, wherein Z is selected from a monoclonal antibody designated as AICD2.M1, obtainable from hybridoma cell line 1 H 10 (DSM ACC2118) and another monoclonal antibody designated as AICD2.M2, obtainable from hybridoma cell line 7 D 3 (DSM ACC2119).

2. A bispecific molecule according to claim 1, wherein the antibody determinant X derives from the monoclonal antibodies MAb 425 (ATCC HB 9629) or MAb 361 (ATCC HB 9325).

3. A bispecific molecule according to claim 1 or 2, wherein said bispecific molecule is a F(ab')2 molecule.

4. A bispecific F(ab')2 molecule according to any of claims 1 to 3, wherein the combination of determinants X and Z is one of the following:

   X is derived from Mab 425 and Z is derived from Mab AICD2.M1;
   X is derived from Mab 425 and Z is derived from Mab AICD2.M2;
   X is derived from Mab 361 and Z is derived from Mab AICD2.M1;
   X is derived from Mab 361 and Z is derived from Mab AICD2.M2.

5. A method for preparing a bispecific molecule specified in Claims 3 or 4 by enzymatical conversion of two different monoclonal antibodies, each comprising two identical L (light chain)-H (heavy chain) half molecules and linked by one or more disulfide bonds, into two F(ab')2 molecules, splitting each F(ab')2 molecule under reducing conditions into the Fab' thiols, derivatizing one of these Fab' molecules of each antibody with a thiol activating agent and combining an activated Fab' molecule bearing tumor specificity with one non-activated Fab' molecule bearing leucocyte specificity or vice versa in order to obtain the desired bispecific antibody F(ab')2 fragment, characterized in that monoclonal antibody AICD2.M1 or AICD2.M2 is used as antibody recognizing leucocyte antigen.

6. A method of preparing a bispecific molecule according to Claim 5, wherein monoclonal antibody MAb 425 or MAb 361 is used as antibody recognizing tumor cell antigen.

7. The monoclonal antibody AICD2.M1 recognizing antigen CD2, obtainable by isolation from cell line 1 H 10 deposited under accession no. **DSM ACC2118.**

8. The monoclonal antibody AICD2.M2 recognizing antigen CD2, obtainable by isolation from cell line 7 D 3 deposited under accession no. **DSM ACC2119.**

9. A pharmaceutical formulation comprising at least one bispecific molecule according to any of the claims 1 to 4 together with a pharmaceutically acceptable carrier, excipient or diluent.

10. A pharmaceutical kit of parts comprising

   (i) a first pharmaceutical formulation (I) according to claim 9 comprising a bispecific molecule according to claim 1, wherein Z is derived from AICD2.M2, and

(ii) a second seperate pharmaceutical formulation (II) comprising MAb AICD2.M1 or a Fv or a F(ab')2 fragment thereof or a bispecific molecule according to claim 1, wherein Z is derived from MAb AICD2.M1.

11. A pharmaceutical kit of parts comprising

(i) a first pharmaceutical formulation (I) according to claim 9 comprising a bispecific molecule according to claim 1, wherein Z is derived from AICD2.M1, and
(ii) a second seperate pharmaceutical formulation (II) comprising MAb AICD2.M2 or a Fv or a F(ab')2 fragment thereof or a bispecific molecule according to claim 1, wherein Z is derived from MAb AICD2.M2.

12. A pharmaceutical kit of parts accoeding to claim 11, wherein formulation (I) comprises the bispecific F(ab')2 molecule in which X is derived from MAb 425 and Z is derived from MAb AICD2.M1, or in which X is derived from MAb 361 and Z is derived from MAb AICD2.M1, and formulation (II) comprises a F(ab')2 fragment of MAb AICD2.M2.

13. A method for purging of tumor cells *ex vivo* in autologous bone marrow transplantation by means of a pharmaceutical kit of parts according to one of the claims 10 or 12, optionally in the presence of additional T-lymphocytes, whereby to begin with the cells are treated with formulation (I) and after that with formulation (II), optionally followed by a conventional purification step.

14. Use of a bispecific antibody fragment according to one of the claims 1 to 4 for preparing a drug for the treatment of human tumors.

**Patentansprüche**

1. Bispezifisches Molekül, das sich für die Lyse von Tumorzellen eignet, mit Antikörperdeterminanten X und Z, wobei X für einen Epitop auf einem Tumorantigen und Z für einen Epitop auf dem Antigen CD2 spezifisch ist, wobei Z aus der Gruppe umfassend einen monoklonalen Antikörper mit der Bezeichnung AICD2.M1, der aus der Hybridomzellinie 1 H 10 (DSM ACC2118) erhältlich ist, und einen weiteren monoklonalen Antikörper mit der Bezeichnung AICD2.M2, der aus der Hybridomzellinie 7 D 3 (DSM ACC2119) erhältlich ist, ausgewählt wird.

2. Bispezifisches Molekül nach Anspruch 1, wobei die Antikörperdeterminante X von den monoklonalen Antikörpern MAb 425 (ATCC HB 9629) oder MAb 361 (ATCC HB 9325) stammt.

3. Bispezifisches Molekül nach Anspruch 1 oder 2, wobei es sich bei dem bispezifischen Molekül um ein F(ab')2-Molekül handelt.

4. Bispezifisches F(ab')2-Molekül nach einem der Ansprüche 1 bis 3, wobei die Kombination der Determinanten X und Z eine der folgenden Kombinationen darstellt:

X stammt von Mab 425 und Z von Mab AICD2.M1;
X stammt von Mab 425 und Z von Mab AICD2.M2;
X stammt von Mab 361 und Z von Mab AICD2.M1;
X stammt von Mab 361 und Z von Mab AICD2.M2.

5. Verfahren zur Herstellung eines bispezifischen Moleküls nach Anspruch 3 oder 4 durch die enzymatische Umwandlung zweier unterschiedlicher monoklonaler Antikörper, die jeweils zwei identische L- (light chain)-H- (heavy chain)-Halbmoleküle umfassen und durch eine oder mehrere Disulfidbrücken verbunden sind, in zwei F(ab')2-Moleküle, Spaltung jedes F(ab')2-Moleküls unter reduzierenden Bedingungen in die Fab'-Thiole, Derivatisierung eines dieser Fab'-Moleküle jedes Antikörpers mit einem thiolaktivierenden Mittel, und Kombination eines aktivierten Fab'-Moleküls mit Tumorspezifität mit einem nicht aktivierten F(ab')-Molekül mit Leukozytenspezifität oder umgekehrt zwecks Erhalt des gewünschten bispezifischen Antikörper-F(ab')2-Fragments, dadurch gekennzeichnet, daß der monoklonale Antikörper AICD2.M1 oder AICD2.M2 als antikörpererkennendes Leukozytenantigen verwendet wird.

6. Verfahren zur Herstellung eines bispezifischen Moleküls nach Anspruch 5, wobei als antikörpererkennendes Tumorzellantigen der monoklonale Antikörper MAb 425 oder MAb 361 verwendet wird.

**7.** Der das Antigen CD2 erkennende monoklonale Antikörper AICD2.M1, der durch Isolation aus der unter der Eingangsnummer **DSM ACC2118** hinterlegten Zellinie 1 H 10 erhältlich ist.

**8.** Der das Antigen CD2 erkennende monoklonale Antikörper AICD2.M2, der durch Isolation aus der unter der Eingangsnummer **DSM ACC2119** hinterlegten Zellinie 7 D 3 erhältlich ist.

**9.** Arzneimittelformulierung mit mindestens einem bispezifischen Molekül nach einem der Ansprüche 1 bis 4 sowie einem pharmazeutisch unbedenklichen Träger, Grundstoff oder Streckmittel.

**10.** Arzneimittelkit aus Teilen umfassend

   (i) eine erste Arzneimittelformulierung (I) nach Anspruch 9 mit einem bispezifischen Molekül nach Anspruch 1, wobei Z von AICD2.M2 stammt, sowie
   (ii) eine separate zweite Arzneimittelformulierung (II) mit dem MAb AICD2.M1 oder dessen Fv- oder F(ab')2-Fragment oder einem bispezifischen Molekül nach Anspruch 1, wobei Z von MAb AICD2.M1 stammt.

**11.** Arzneimittelkit aus Teilen umfassend

   (i) eine erste Arzneimittelformulierung (I) nach Anspruch 9 mit einem bispezifischen Molekül nach Anspruch 1, wobei Z von AICD2.M1 stammt, sowie
   (ii) eine separate zweite Arzneimittelformulierung (II) mit dem MAb AICD2.M2 oder dessen Fv- oder F(ab')2-Fragment oder einem bispezifischen Molekül nach Anspruch 1, wobei Z von MAb AICD2.M2 stammt.

**12.** Arzneimittelkit aus Teilen gemäß Anspruch 11, wobei die Formulierung (I) das bispezifische F(ab')2-Molekül umfaßt, bei dem X von MAb 425 und Z von MAb AICD2.M1 stammt, oder bei dem X von MAb 361 und Z von MAb AICD2.M1 stammt, und die Formulierung (II) ein F(ab')2-Fragment von MAb AICD2.M2 umfaßt.

**13.** Verfahren zur Entfernung von Tumorzellen *ex vivo* bei der autologen Knochenmarkstransplantation unter Verwendung eines Arzneimittelkits mit Teilen nach Anspruch 10 oder 12, gegebenenfalls in Gegenwart zusätzlicher T-Lymphozyten, wobei die Zellen zuerst mit der Formulierung (I) und anschließend mit der Formulierung (II) behandelt werden, woran sich gegebenenfalls ein traditioneller Reinigungsschritt anschließt.

**14.** Verwendung eines bispezifischen Antikörperfragments nach einem der Ansprüche 1 bis 4 zur Herstellung eines Medikaments zur Behandlung menschlicher Tumore.

**Revendications**

**1.** Molécule bispécifique, utile pour la lyse des cellules tumorales, comprenant les déterminants d'anticorps X et Z, parmi lesquels Z est spécifique d'un épitope sur un antigène tumoral, et Z est spécifique d'un épitope de l'antigène CD2, où Z est choisi parmi un anticorps monoclonal désigné par AICD2.M1, que l'on peut obtenir à partir de la lignée cellulaire d'hybridome 1 H 10 (DSM ACC2118) et un autre anticorps monoclonal désigné par AICD2.M2, que l'on peut obtenir à partir de la lignée cellulaire d'hybridome 7 D 3 (DSM ACC2119).

**2.** Molécule bispécifique selon la revendication 1, dans laquelle le déterminant d'anticorps X provient des anticorps monoclonaux MAb 425 (ATCC HB 9629) ou MAb 361 (ATCC HB 9325).

**3.** Molécule bispécifique selon la revendication 1 ou 2, où ladite molécule bispécifique est une molécule de F(ab')2.

**4.** Molécule de F(ab')2 bispécifique selon l'une quelconque des revendications 1 à 3, dans laquelle la combinaison des déterminants X et Z est l'une des suivantes :

   X provient de Mab 425 et Z provient de Mab AICD2.M1 ;
   X provient de Mab 425 et Z provient de Mab AICD2.M2 ;
   X provient de Mab 361 et Z provient de Mab AICD2.M1 ;
   X provient de Mab 361 et Z provient de Mab AICD2.M2.

**5.** Procédé de préparation d'une molécule bispécifique spécifiée dans les revendications 3 ou 4 par transformation enzymatique de deux anticorps monoclonaux différents, comprenant chacun deux demi-molécules L (chaîne légère)-H (chaîne lourde) identiques et réunies par une ou plusieurs liaisons disulfure, en deux molécules de F(ab')2, division de chaque molécule de F(ab')2 dans des conditions réductrices en les thiols de Fab', transformation en dérivés de l'une de ces molécules de Fab' de chaque anticorps avec un agent activateur de thiol et combinaison d'une molécule de Fab' activée portant une spécificité tumorale avec une molécule de Fab' non activée portant une spécificité leucocytaire ou vice versa afin d'obtenir le fragment d'anticorps bispécifique F(ab')2 désiré, caractérisé en ce que l'anticorps monoclonal AICD2.MI ou AICD2.M2 est utilisé comme anticorps reconnaissant l'antigène leucocytaire.

6. Procédé de préparation d'une molécule bispécifique selon la revendication 5, dans lequel on utilise l'anticorps monoclonal MAb 425 ou MAb 361 comme anticorps reconnaissant l'antigène cellulaire tumoral.

7. Anticorps monoclonal AICD2.M1 reconnaissant l'antigène CD2, que l'on peut obtenir par isolement à partir de la lignée cellulaire 1 H 10 déposée sous le n° d'accès **DSM ACC2118.**

8. Anticorps monoclonal AICD2.M2 reconnaissant l'antigène CD2, que l'on peut obtenir par isolement à partir de la lignée cellulaire 7 D 3 déposée sous le n° d'accès **DSM ACC2119.**

9. Formulation pharmaceutique comprenant au moins une molécule bispécifique selon l'une quelconque des revendications 1 à 4 avec un support, excipient ou diluant pharmaceutiquement acceptable.

10. Nécessaire de pièces pharmaceutiques comprenant

(i) une première formulation pharmaceutique (I) selon la revendication 9 comprenant une molécule bispécifique selon la revendication 1, dans laquelle Z provient d'AICD2.M2, et
(ii) une seconde formulation pharmaceutique séparée (II) comprenant MAb AICD2.M1 ou un de ses fragments Fv ou F(ab')2 ou une molécule bispécifique selon la revendication 1, où Z provient de MAb AICD2.M1.

11. Nécessaire de pièces pharmaceutiques comprenant

(i) une première formulation pharmaceutique (I) selon la revendication 9 comprenant une molécule bispécifique selon la revendication 1, où z provient d'AICD2.M1, et
(ii) une seconde formulation pharmaceutique séparée (II) comprenant MAb AICD2.M2 ou un de ses fragments Fv ou F(ab')2 ou une molécule bispécifique selon la revendication 1, où Z provient de MAb AICD2.M2.

12. Nécessaire de pièces pharmaceutiques selon la revendication 11, dans lequel la formulation (I) comprend la molécule F(ab')2 bispécifique dans laquelle X provient de MAb 425 et Z provient de MAb AICD2.M1, ou bien dans laquelle X provient de MAb361 et Z provient de MAb AICD2.M1, et la formulation (II) comprend un fragment F(ab')2 de MAb AICD2.M2.

13. Procédé pour purger les cellules tumorales *ex vivo* dans une greffe autologue de moelle osseuse au moyen d'un nécessaire de pièces pharmaceutiques selon l'une des revendications 10 ou 12, facultativement en présence de lymphocytes T supplémentaires, dans lequel pour commencer on traite les cellules avec la formulation (I) puis avec la formulation (II), puis on procède facultativement à une étape de purification classique.

14. Utilisation d'un fragment d'anticorps bispécifique selon l'une des revendications 1 à 4 pour préparer un médicament pour le traitement des tumeurs humaines.

Fig. 1

EP 0 637 593 B1

Ellman's Reagent

Fig. 2

Fig. 3

EP 0 637 593 B1

16

mol/l [log]

Fig. 4

Fig. 5

Fig. 6 A

Fig. 6 B

Fig. 7 A

Fig. 7 B